# EUROPEAN PATENT APPLICATION

(11) **EP 1 587 100 A1**
(43) Date of publication of application: **19.10.2005**
(21) Application number: 03782941.3
(22) Date of filing: 26.12.2003
(51) Int. Cl.: G11B 20/12, G11B 27/00, G10H 1/00

(54) **DATA RECORDING MEDIUM, RECORDING METHOD AND RECORDER, REPRODUCING METHOD AND REPRODUCER, AND DATA TRANSMITTING METHOD AND TRANSMITTER**

(30) Priority: 21.01.2003 JP 2003012509
(71) Applicant: SONY CORPORATION, Tokyo 141-0001 (JP)
(72) Inventor: SAKO, Yoichiro C/O SONY CORPORATION, Shinagawa-ku, Tokyo 141-0001 (JP)
(74) Representative: Müller - Hoffmann & Partner
(86) International application number: PCT/JP2003/016939
(87) International publication number: WO 2004/066299

(57) **Abstract**

PCM audio data is recoded in a first session P1 as a first region on the inner periphery side of an optical disc Ma. Living body information is recorded in a second session P2 as a second region on the outer periphery side. The living body information is information about a living body of a music performer, an actor (actress), audience, or the like. AV information and living body information recorded on the optical disc are temporally synchronized. PCM audio data and living body information are reproduced from respective sessions. The tempo, level, and so forth of the reproduction PCM audio signal are controlled in accordance with the living body information. Thus, a listener who listens to a sound of the PCM audio signal controlled in accordance with the living body information can feel a live power.

## Description

### Technical Field

The present invention relates to a data recording medium, a data recording method, a data recording apparatus, a data reproducing method, a data reproducing apparatus, a data transmitting method, and a data transmitting apparatus that are used to record, transmit, and reproduce at least one of audio information and video information.

### Background Art

Nowadays, packaged mediums such as a CD (Compact Disc), a DVD (Digital Versatile Disc), and a video are common. Concert discs and concert videos have been circulated. When contents are reproduced from such a medium, since handclaps of audience are recorded thereon, the listener can feel atmosphere of the concert hall to some extent.

Thus far, a musical performance controlling apparatus that allows the user to comfortably listen to music with a 1/f noise, which is a rhythm of a human's living body, added to original music has been proposed. A technology for generating a noise control signal in accordance with situation of the living body of the listener due to the fact that situation and condition of a human's living body vary time by time and editing the tempo of musical performance of an audio program in accordance with the noise control signal is disclosed in a patent related art reference (Japanese Patent Laid-Open Publication No. HEI 10-79130).

According to the technology of the patent related art reference 1, the tempo of musical performance is controlled in accordance with living body information about health of the listener. Thus, even if comfortable music can be reproduced for the listener, since living body information of a music performer, an actor, and an actress is not recorded, the listener cannot feel live power from the reproduced data.

Thus, an object of the present invention is to provide a data recording medium, a data recording method, a data recording apparatus, a data reproducing method, a data reproducing apparatus, a data transmitting method, and a data transmitting apparatus that allow the listener to feel live power such as breathing and excitement of a music performer, an actor, an actress, and audience along with audio information and video information.

### Disclosure of the Invention

To solve the foregoing problem, claim 1 of the present invention is a data recording medium having a first region and a second region, at least one of audio data and video data being recorded in the first region, living body information in relation to the data being recorded in the second region.

Claim 13 of the present invention is a recording method for recording at least one of audio data and video data to a first region and living body information in relation to the data to a second region.

Claim 14 of the present invention is a recording apparatus for recording at least one of audio data and video data to a first region and living body information in relation to the data to a second region.

Claim 15 of the present invention is a reproducing method, comprising the steps of:
reading data from a data recording medium having a first region and a second region, at least one of audio data and video data being recorded in the first region, living body information in relation to the data being recorded in the second region; and
reproducing the data that has been read and reproducing the living body information with one of a sound, a picture, and a vibration.

Claim 16 of the present invention is a reproducing method, comprising the steps of:
reading data from a data recording medium having a first region and a second region, at least one of audio data and video data being recorded in the first region, living body information in relation to the data being recorded in the second region; and
controlling the reproduction of the data recorded in the first region corresponding to the living body information.

Claim 17 of the present invention is a reproducing apparatus, comprising:
reading means for reading data from a data recording medium having a first region and a second region, at least one of audio data and video data being recorded in the first region, living body information in relation to the data being recorded in the second region;
reproducing means for reproducing the data that has been read by the reading means; and
reproduction controlling means for reproducing the data that is recorded in the first region and reproducing the living body information, which is in relation to the data, with one of a sound, a picture, and a vibration.

Claim 18 of the present invention is a reproducing apparatus, comprising:
reading means for reading data from a data recording medium having a first region and a second region, at least one of audio data and video data being recorded in the first region, living body information in relation to the data being recorded in the second region;
reproducing means for reproducing the data that has been read by the reading means; and
controlling means for controlling the reproduction of the data recorded in the first region corresponding to the living body information.

Claim 21 of the present invention is a data transmitting method, comprising the steps of:
dividing at least one of audio data and video data in a predetermined region;
generating block data composed of the divided data and living body information corresponding to the predetermined region; and
transmitting the generated block data.

Claim 30 of the present invention is a data transmitting apparatus, comprising:
dividing means for dividing at least one of audio data and video data in a predetermined region;
generating means for generating block data composed of the divided data divided by the dividing means and living body information corresponding to the predetermined region; and
transmitting means for transmitting the block data generated by the generating means.

Claim 31 of the present invention is a recording method, comprising the steps of:
dividing at least one of audio data and video data in a predetermined region;
generating block data composed of the divided data and living body information corresponding to the predetermined region; and
recording the generated block data.

Claim 32 of the present invention is a recording apparatus, comprising:
data dividing means for dividing at least one of audio data and video data in a predetermined region;
generating means for generating block data composed of the divided data and living body information corresponding to the predetermined region; and
recording means for recording the block data generated by the generating means to a recording medium.

Claim 33 of the present invention is a recording medium on which at least one of audio data and video data divided in a predetermined region and living body information corresponding to the predetermined region are recorded as a block.

Claim 34 of the present invention is a reproducing method, comprising the steps of:
receiving a block composed of data and living body information, the data being at least one of audio data and video data; and
reproducing the data and reproducing the living body information with one of a sound, a picture, and a vibration.

Claim 35 of the present invention is a reproducing method, comprising the steps of:
receiving block data composed of data and living body information, the data being at least one of audio data and video data; and
controlling the block data corresponding to the living body information and reproducing the block data.

Claim 36 of the present invention is a reproducing method, comprising the steps of:
reading block data from a recording medium, the block being composed of data and living body information, the data being at least one of audio data and video data divided in a predetermined region, the living body information corresponding to the predetermined region; and
reproducing the data and reproducing the living body information with one of a sound, a picture, and a vibration.

Claim 37 of the present invention is a reproducing method, comprising the steps of:
reading block data composed of data and living body information, the data being at least one of audio data and video data divided in a predetermined region, the living body information corresponding to the predetermined region; and
controlling the data corresponding to the living body information and reproducing the data.

Claim 38 of the present invention is a reproducing apparatus, comprising:
receiving means for receiving block data composed of data and living body information, the data being at least one of audio data and video data divided in a predetermined region, the living body information corresponding to the predetermined region; and
reproducing means for reproducing the data and reproducing the living body information with one of a sound, a picture, and a vibration.

Claim 39 of the present invention is a reproducing apparatus, comprising:
receiving means for receiving block data composed of data and living body information, the data being at least one of audio data and video data divided in a predetermined region, the living body information corresponding to the predetermined region;
reproducing means for reproducing the block data; and
controlling means for reproducing the data corresponding to the living body information.

Claim 40 of the present invention is a reproducing apparatus, comprising:
reading means for reading block data from a recording medium, the block data being composed of data and living body information, the data being at least one of audio data and video data divided in a predetermined region, the living body information corresponding to the predetermined region; and
reproducing means for reproducing the data and reproducing the living body information with one of a sound, a picture, and a vibration.

Claim 41 of the present invention is a reproducing apparatus, comprising:
reading means for reading block data from a recording medium, the block data being composed of data and living body information, the data being at least one of audio data and video data divided in a predetermined region, the living body information corresponding to the predetermined region;
reproducing means for reproducing the data; and
controlling means for controlling the reproduction of the data corresponding to the living body information.

### Brief Description of Drawings

Fig. 1A to Fig. 1F are schematic diagrams describing several examples of data recording mediums according to an embodiment of the present invention.
Fig. 2 is a block diagram showing a structure for detecting a breath signal as a living body signal.
Fig. 3 is a block diagram showing a structure for detecting a heartbeat signal as a living body signal.
Fig. 4 is a block diagram showing an example of a structure of a data recording apparatus that uses physically divided regions according to the present invention.
Fig. 5 is a block diagram describing an example of a recording apparatus.
Fig. 6 is a block diagram showing an example of the structure of the data recording apparatus that uses physically divided regions according to the present invention.
Fig. 7 is a block diagram showing an example of a structure of a reproducing apparatus according to the present invention.
Fig. 8 is a block diagram showing an example of a structure of a reproduction controlling apparatus in accordance with reproduced living body information.
Fig. 9A and Fig. 9B are schematic diagrams showing an example of a data structure describing another embodiment of the present invention.
Fig. 10 is a block diagram showing an example of a structure of a data recording apparatus according to another embodiment of the present invention.
Fig. 11 is a block diagram showing an example of a structure of a data reproducing apparatus according to another embodiment of the present invention.
Fig. 12 is a block diagram showing an example of a structure of a data transmitting apparatus according to another embodiment of the present invention.
Fig. 13 is a block diagram showing an example of a data receiving apparatus according to another embodiment of the present invention.

### Best Modes for Carrying out the Invention

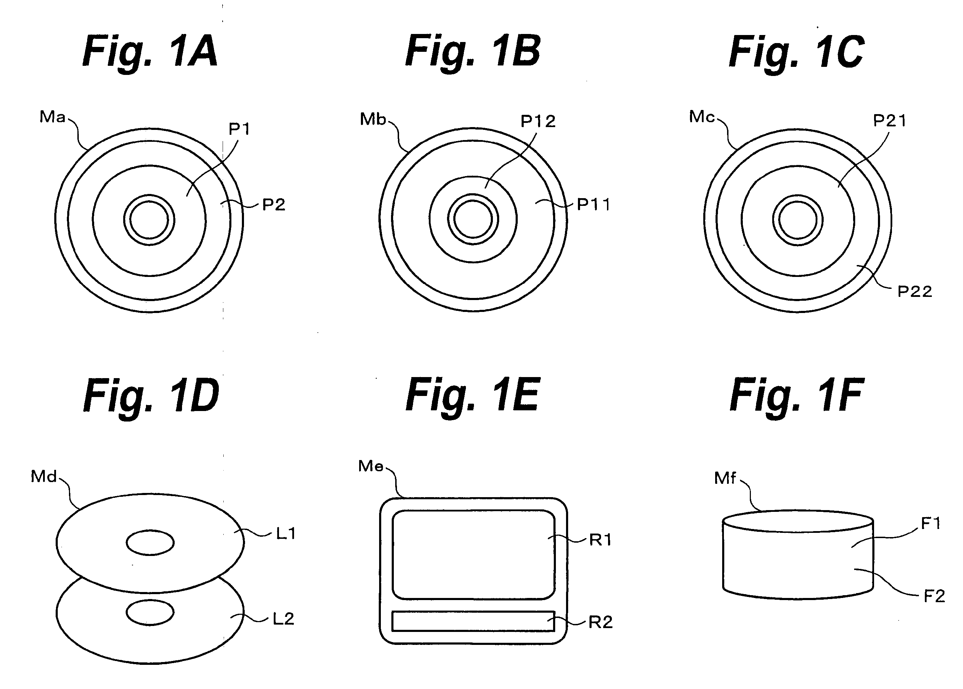

Next, an embodiment of the present invention will be described. Fig. 1A to Fig. 1F show a plurality of examples of data recoding mediums according to the present invention. In Fig. 1A, reference letter Ma represents an example of a CD formatted optical disc according to the present invention. PCM audio data is recorded in a first session P1 as a first region on the inner periphery side. Living body information is recorded in a second session P2 as a second region on the outer periphery side. These sessions each have a lead-in area, a program area, and a lead-out area successively formed from the inner periphery side to the outer periphery side. Data that is recorded on the recording medium is based on the CD-format standard. For example, data recorded on the recording medium is based on the CD-EXTRA format.

In Fig. 1B, reference letter Mb represents an example of a CD-ROM formatted optical disc according to the present invention. Compressed AV information is recorded in a session P11 formed on the outer periphery side. Living body information is recorded in a session P12 on the inner periphery side. AV information is compressed in accordance with for example MPEG2 (Moving Picture Experts Group Phase 2). Audio information is compressed in accordance with ATRAC (Adaptive Transfer Acoustic Coding), MP3 (MPEG1 Audio Layer III), or the like. Data recorded on the optical disc is based on the CD-ROM format standard (so-called "Yellow Book"). Alternatively, audio data may be a one-bit digital audio signal of which large and low levels of a signal are represented by thin and dense states, respectively.

In Fig. 1C, reference letter Mc represents an example of a DVD according to the present invention. Video data that has been compressed in accordance with MPEG2 is recorded in a session on the inner periphery side. Audio data that has been compressed in accordance with AC-3 and audio data that has been compressed in accordance with AC-3 are recorded in a session on the outer periphery side. Data of living body information is recorded in a session on the outer periphery side.

In Fig. 1D, reference letter Md represents a single-sided or double-sided two-layered DVD. Video data that has been compressed in accordance with MPEG2 and audio data that has been compressed in accordance with AC-3 are recorded on a recording layer L1. Data of living body information is recoded on a recording layer L2. Data recorded on each of the recording layers is based on the DVD format standard.

In Fig. 1E, reference numeral Me represents an example of an optical card according to the present invention. AV information is recorded in a first recording area R1 of the optical card. Data of living body information is recorded in a second recording area of the optical card. When necessary, AV information that is recorded in each of the recording areas is compressed.

In Fig. 1F, reference letter Mf represents an example of a hard disk drive according to the present invention. AV information is recorded as a first file F1 to the hard disk drive. Data of living body information is recorded as a second file F2 to the hard disk drive. When necessary, AV information recorded in the file F1 is compressed. Video information may be compressed in accordance with the DV (digital Video) format, which is used for a home-use digital VTR, other than MPEG2. Although the mediums shown in Fig. 1A to Fig. 1E have physically divided regions, the hard disk drive Mf has logically dived regions.

When audio data is handled, living body information is information about a living body of one or a plurality of music performers (a singer, a music performer of an instrument, and so forth) and listeners such as audience of a concert hall. When video information is handled, living body information is information about living bodies of actors and actresses or information of living bodies of audiences who watch video information that is recorded. These audiences are generally referred to as performer. AV information and living body information recorded on a data recording medium are temporally synchronized. In other words, living body information of the performer is different from living body information of a viewer who reproduces AV information from a data recording medium and watches the reproduced AV information.

In addition to the recording mediums shown in Fig. 1, the present invention can be applied to other data recording mediums such as a memory card, an organic compound memory, and a magnetic card.

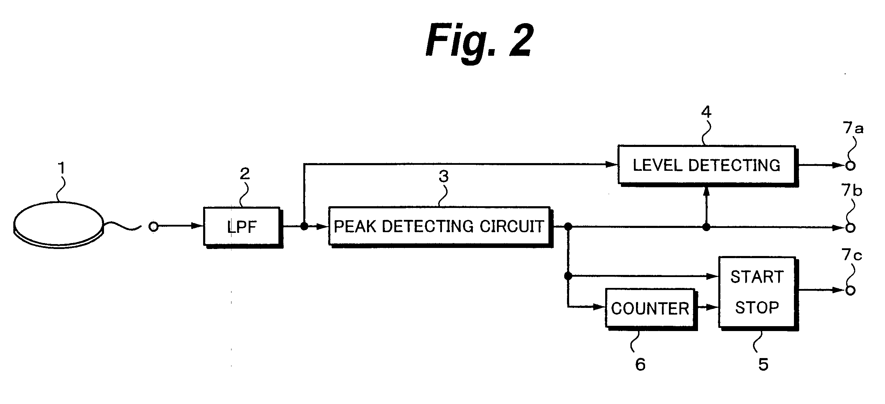

Living body information of the performer is at least one of a body motion, a biochemical reaction, a brain wave, a magnetoencephalography, an electromyogram, a body surface temperature, perspiration of a skin, a skin resistance, a pulsation, breath, a micro vibration, an electrocardiogram, a heartbeat, and a blood pressure. Fig. 2 shows an example of a structure for detecting for example a breath of the performer. An air speed and an air flow amount of breathing gas are detected by an air speed meter and an air flow amount meter that are placed in an air flow path. In the example shown in Fig. 2, to measure motion of a chest easily and inexpensively, it is electrically measured.

In Fig. 2, reference numeral 1 represents a sensor that detects motion of a chest in accordance with a breathing exercise. Breathing is measured with impedance of a living body that varies. In this case, four electrodes are placed on the chest. A current is supplied to two outer electrodes placed on the chest. A potential is detected from two inner electrodes placed on the chest. Alternatively, a sensor such as a distortion gauge that detects the variation of the volume of the chest in accordance with the breathing exercise may be placed on the chest.

The sensor 1 generates a detection signal whose amplitude varies in accordance with the breathing exercise. The detection signal is supplied to a low pass filter 2. The low pass filter 2 removes an unnecessary signal component such as noise from the detection signal. It is preferred that the signal generated by the sensor 1 is wirelessly transmitted to the low pass filter 12 so as to prevent a cable from disturbing motion of the performer. An output signal of the low pass filter 2 is supplied to a peak detecting circuit 3 and a level detecting circuit 4. The peak detecting circuit 3 detects a plus peak value and a minus side. The detection signal is nearly a sine wave of which a period from the plus peak value to the minus peak value is an inhalation period and a period from the minus peak value to the plus peak value is an exhalation period.

The detection signal is supplied from the peak detecting circuit 3 to the level detecting circuit 4. The level detecting circuit 4 detects the level of the peak value of the detection signal. The signal that represents the detected level is obtained from an output terminal 7a. The signal obtained from the output terminal 7a represents the depth of breathing. The detection signal of the peak detecting circuit 3 is obtained from an output terminal 7b. A pulse signal at timings of a plus peak value and a minus peak value is obtained as a detection output from the output terminal 7b. The detection output of the output terminal 7b is used as a timing signal.

In addition, the output signal of the peak detecting circuit 3 is supplied as a start signal to a timer 5. In addition, the output signal of the peak detecting circuit 3 is input to a counter 6. An output of the counter 6 is supplied as a stop signal to the timer 5. When the counter 6 has counted up n peaks of the signal, the counter 6 outputs a stop signal to the timer 5. Thus, the timer 5 outputs a detection signal every n peak values. The detection signal that the timer 5 outputs is obtained as a breath interval output from an output terminal 7c. In such a manner, a breath depth signal, a breath timing signal, and a breath interval signal are output from the outputs 7a, 7b, and 7c, respectively.

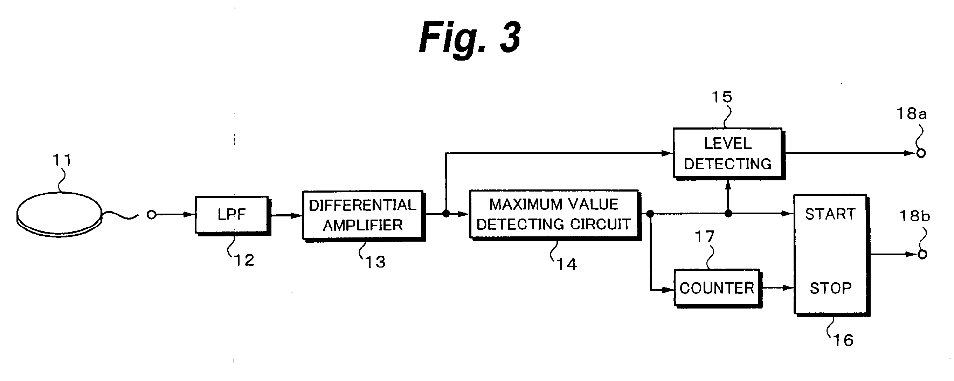

Fig. 3 shows an example of a structure for measuring a heartbeat as living body information. For example, a current supplying electrode is placed around the neck of the performer. A detection electrode is placed on the chest of the performer. A high frequency constant current is supplied to the current supplying electrode. In Fig. 3, reference numeral 11 represents a detection electrode. When necessary, a current detected by the detection electrode 11 is converted into a signal whose voltage or impedance varies. The signal is supplied to a low pass filter 12. It is preferred that the signal that is obtained from the detection electrode 11 is wirelessly transmitted to the low pass filter 12. The low pass filter 12 removes an unnecessary component such as noise from the detection signal. Alternatively, movement of the chest may be detected with a pressure sensor placed around the heart of the performer.

An output signal of the low pass filter 12 is supplied to a differential amplifier 13. The differential amplifier 13 linearly differentiates the detection signal. The differentiated signal is supplied to a maximum value detecting circuit 14 and a level detecting circuit 15. The maximum value detecting circuit 14 generates a pulse signal at a timing of which the level of the differentiated signal becomes the maximum. The pulse signal is supplied to the level detecting circuit 15. The level detecting circuit 15 detects the level of the differentiated signal as a timing of the pulse signal and outputs the pulse signal to an output terminal 18a. An output signal obtained from the output terminal 18a is a detection signal that represents the intensity of the heartbeat of the performer.

The output of the maximum value detecting circuit 14 is supplied as a start signal to a timer 16. A counter 17 counts up the output of the maximum value detecting circuit 14. When the counter 17 has counted up the maximum value n times, the counter 17 outputs a stop signal to the timer 16. An output signal of the timer 16 is obtained from an output terminal 18b. A detection signal that is obtained from the output terminal 18b represents an interval of heartbeat of the performer.

A blood pressure as another living body information is measured with a cuff that has a sensor that detects variation of a cuff pressure. Waves of electrocardiogram and electromyogram, brain wave, and so forth are measured with electrodes that are placed on the surface of the living body. The electrodes detect an electric signal of the living body. Electricity of the living body is a membrane potential that an exciting cell such as a nerve or a muscle cell generates. The membrane potential depends on variation of permeability of a cell membrane against ions. An impedance such as resistance of a skin can be measured with an electric charge.

A flow of ions due to an electric activity of the living body induces a potential on the outer surface of the body and a magnetic field outside the body. For example, a magnetic field takes place in the brain in accordance with an electric activity in the brain. The magnetic field in the brain can be measured with a magnetic flux gauge having a high sensitivity. The surface temperature of the body can be measured with a temperature detecting sensor at a predetermined portion of the body. A micro vibration is a weak vibration that takes place on the outer surface of the skin. The amplitude of the micro vibration varies in accordance with an emotion, excitement, and so forth of a human. The micro vibration can be detected with a pressure sensor.

In addition, pulsation as another living body information can be measured using ultrasonic waves. In addition, motion of the body can be used as living body information. A light emitting device (that blinks at a predetermined period) is disposed at least one position of the body to be measured. The light emitting device is photographed. The position of the light emitting device is detected on a two-dimensional photographic plane. As a result, the motion of the body can be measured. The timing at which the motion of the body becomes the maximum is measured. Timing is controlled so that when the motion of the body becomes the maximum, reproduction is started.

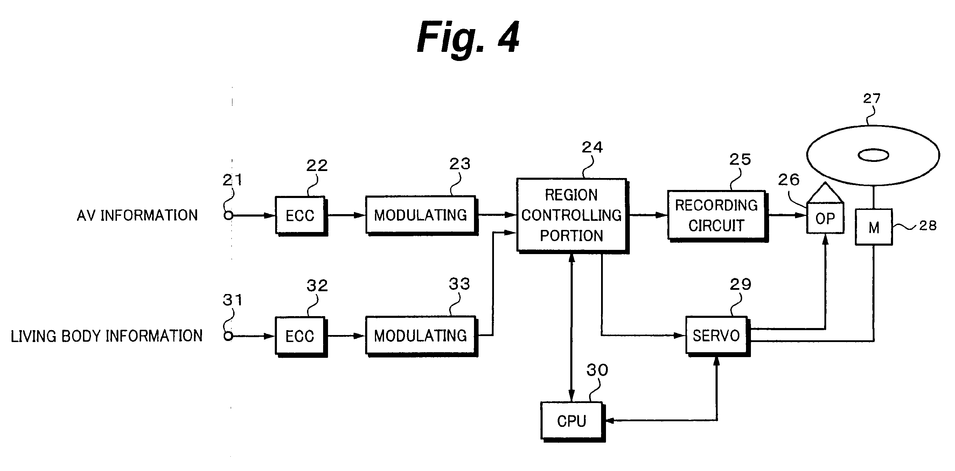

Next, with reference to Fig. 4, a recording apparatus that reproduces data from the foregoing recording medium for example an optical disc Ma having two sessions will be described. AV information for example an audio PCM signal is supplied to an input terminal represented by reference numeral 21. The audio PCM signal is supplied to a modulating circuit 23 through an error correction code encoder 22.

Living body information of the performer, for example a breath signal obtained by the structure shown in Fig. 2, is supplied to an input terminal represented by reference numeral 31. The audio PCM signal that is recorded and the breath signal are temporally synchronized. The audio PCM signal is supplied to a modulating circuit 33 through an error correction code encoder 32. The error correction code encoders 22 and 32 are CIRC (Cross Interleaved Reed-Solomon Code) encoders. The encoders 22 and 32 perform an error correction code encoding process for adding error correction parity data to the audio PCM signal and a scrambling process. In other words, the encoders 22 and 32 divide 16 bits of one sample or one word into high order eight bits and low order eight bits as two samples and perform the error correction code encoding process and the scramble process in such a manner that error correction parity data or the like in accordance with for example CIRC is added to each symbol. Modulating circuits 23 and 33 perform a demodulating process in accordance with EFM modulating system (Eight to Fourteen Modulation: EFM).

The modulating circuits 23 and 33 obtain digital signals that have the same format as a CD. Output signals of these modulating circuits 23 and 33 are supplied to a region controlling portion 24. The region controlling portion 24 is controlled by a controller 30 composed of a CPU. The region controlling portion 24 supplies address information (sub code of Q channel) separated from a record signal to a servo circuit 29. The address information contains an absolute address corresponding to a record position of the disc. While monitoring addresses of data that is recorded, the controller 30 controls the region controlling portion 24 so that output signals of the modulating circuits 23 and 33 are switched at predetermined addresses. An output signal of the region controlling portion 24 is a record signal. The record signal is supplied to an optical pickup 26 through a recording circuit 25.

The optical pickup 26 records data to a recordable optical disc 27 such as a CD-R (Recordable). The optical disc 27 is placed on a turn table and rotated by a spindle motor 28. The spindle motor 28 is driven and rotated at a constant linear velocity (CLV) under the control of the servo circuit 29.

The servo circuit 29 generates various types of servo drive signals of focus, tracking, thread, and spindle servo drives under the control of an operation command received from the controller 30 and outputs these signals to the spindle motor 28 and the optical pickup 26. The controller 30 controls all the recording apparatus. A display, operation switches, and so forth (not shown) are connected to the controller 30. The optical pickup 26 focus a light beam of a semiconductor laser on a signal side of the optical disc 27 and records data on tracks formed in a concentrically circular shape or a spiral shape on the recordable optical disc 27. All the optical pickup 26 is moved by a thread mechanism.

The optical pickup 26 records data to the recordable optical disc 27 such as a CD-R. The recordable optical disc 27 is placed on the turn table and rotated by the spindle motor 28. The spindle motor 28 is driven and rotated at a constant linear speed (CLV) under the control of the servo circuit 29.

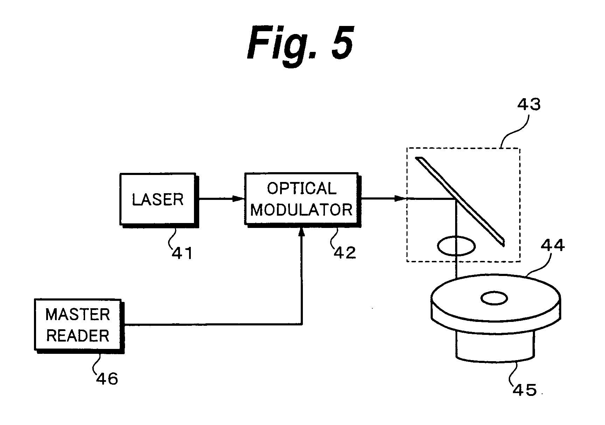

Like the optical disc Ma shown in Fig. 1A, the optical disc 27 has two sessions on which a PCM audio signal and data of living body information are recorded. In addition, TOC (Table Of Contents) information is recorded on the optical disc 27. In addition to information of a conventional CD, address information of a boundary position of a recording region of living body information and a recording region of AV information is recorded on the TOC. The optical disc 27 is used as a master disc. A disc master is produced by a mastering apparatus shown in Fig. 5. A master tape may be used instead of the master disc.

As shown in Fig. 5, the mastering apparatus comprises a laser 41, an optical modulator 42, and an optical pickup 43. The laser 41 is for example a gas laser such as an Ar ion laser, a He-Cd laser, or a Kr ion laser, or a semiconductor laser. The optical modulator 42 is acousto-optical effect type or electro-optical type optical modulator that modulates laser light radiated from the laser 41. The optical pickup 43 is a recording means that has an objective lens or the like that focuses the laser light that passes through the optical modulator 42 on a photoresist side of a disc-shaped glass original 44 on which photoresist as a photosensitive substance is coated.

The optical modulator 42 modulates laser light of the laser 41 in accordance with the record signal. The mastering apparatus radiates the modulated laser light to the glass original 44. As a result, a master on which data is recorded is produced. In addition, the mastering apparatus has servo circuits (not shown) that control the distance between the optical pickup 43 and the glass original 44 so that their distance is kept constant, tracking is controlled, and a rotation driving operation of a spindle motor 45. The glass original 44 is driven and rotated by the spindle motor 45.

A record signal is supplied from a master reader 46 to the optical modulator 42. The master reader 46 reproduces data from the optical disc 27 on which a record signal has been recorded by the recording apparatus described with reference to Fig. 4. With a laser beam modulated by the optical modulator 42, the photoresist on the glass original 44 is exposed. The resultant glass original 44 is developed. Thereafter, an electric plating process is performed for the developed surface of the glass original 44. As a result, a metal master is produced. With the metal master, a mother disc is produced. With the mother disc, a stamper is produced. With the stamper, an optical disc is produced in accordance with compression casting method, injection casting method, or the like.

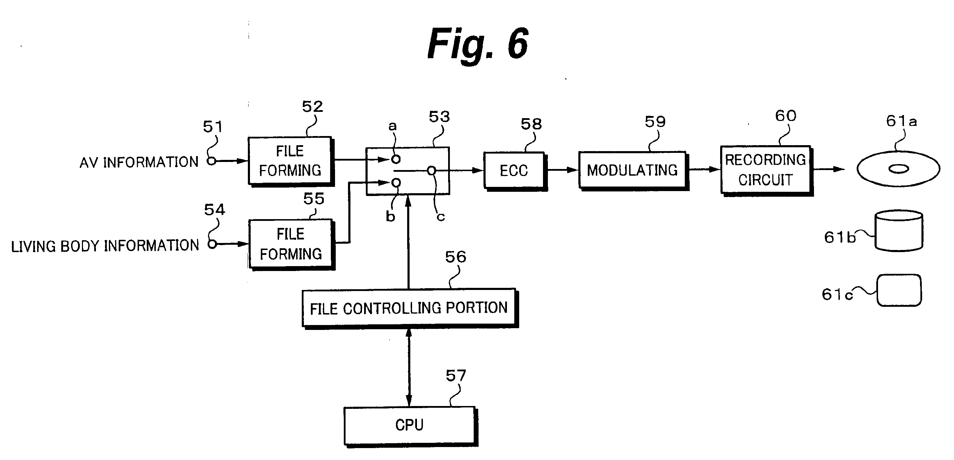

Fig. 6 shows a structure of a recording apparatus that has logically divided regions according to the present invention. AV information is supplied to an input terminal represented by reference numeral 51. The AV information is supplied to one input terminal a of a switch 53 through a file forming portion 52. Living body information for example a breath signal of the performer is supplied to an input terminal represented by reference numeral 54. The breath signal is temporally synchronized with the AV information supplied to the input terminal 51. The living body information is supplied to another input terminal b of the switch 53 through a file forming portion 55. The file forming portions 52 and 55 convert respective input data into respective files.

The switch 53 is controlled in accordance with a control signal supplied from a file controlling portion 56. The file controlling portion 56 is controlled by a controller 57 composed of a CPU. AV information and living body information that are logically divided namely they are converted into different files are selected by the switch 53 and output from an output terminal c thereof.

An output of the switch 53 is supplied to a modulating circuit 59 through an error correction code encoder 58. After an error correction code encoding process is performed for the output data of the switch 53, the encoded data is modulated. The modulated data is supplied to a recording circuit 60. The recording circuit 60 outputs a record signal. The record signal is recorded on an optical disc 61a. In this case, an optical pickup (not shown) is used. When the record signal is recorded to a hard disk drive 61b or an optical card 61c rather than the optical disc 61a, a structure similar to the structure of the signal process for the optical disc 61a can be used.

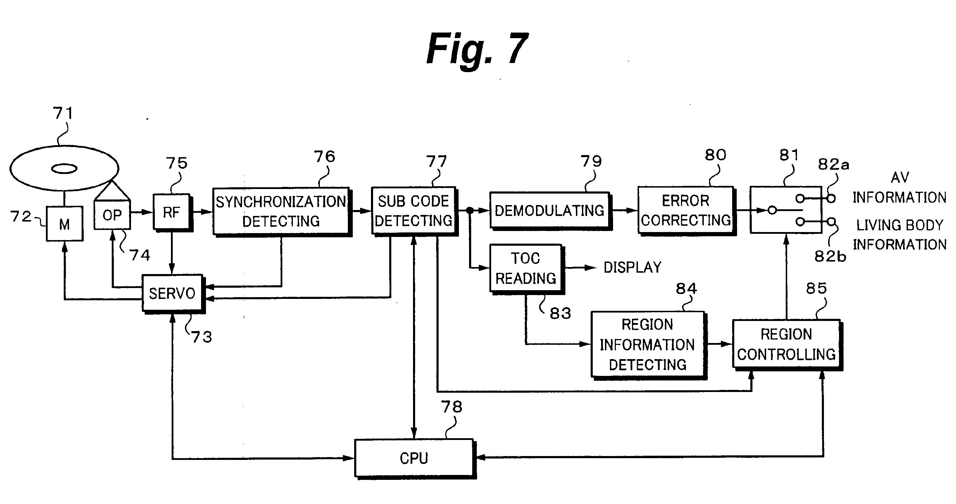

Next, with reference to Fig. 7, a data reproducing apparatus according to an embodiment of the present invention will be described. As described above, a data recording medium from which data is reproduced has physically or logically divided regions for AV information and living body information of the performer that are temporally synchronized. In Fig. 7, reference numeral 71 represents an optical disc having physically divided regions for PCM audio data and living body information like the optical disc 1a shown in Fig. 1A.

An optical disc 71 is placed on a turn table and rotated by a spindle motor 72. The spindle motor 72 is driven and rotated at a constant linear velocity (CLV) under the control of a servo portion 73. The servo portion 73 generates various types of servo drive signals for focus, tracking, thread, and spindle servo drives in accordance with a focus error signal, a tracking error signal, and an operation command received from a controller 78 and outputs these generated signals to the spindle motor 72 and an optical pickup 74. The controller 78 controls all the reproducing apparatus. A display, operation switches, and so forth are connected to the controller. The optical pickup 74 focuses a light beam of a semiconductor laser on a signal side of the optical disc 71 and traces tracks formed in a concentrically circular shape or spiral shape on the optical disc 71. All the optical pickup 74 is moved by a thread mechanism.

An output of the optical pickup 74 is supplied to a synchronization detector 76 through an RF amplifier 75. An output of the synchronization detector 76 is supplied to a sub code detecting circuit 77. The synchronization detector 76 detects a frame synchronization signal for each EFM frame of a reproduction signal. The sub code detecting circuit 77 detects a Q channel of a sub code and detects an address signal of the Q channel.

A servo signal is supplied from the RF amplifier 75 to the servo portion 73. The sub code detected by the sub code detecting circuit 77 is supplied to the servo portion 73, the controller 78, and a region controlling portion 85 that will be described later.

An output signal of the sub code detecting circuit 77 is supplied to for example an EFM demodulator 79 and a TOC reading circuit 83. An output of the demodulator 79 is supplied to an error correcting circuit 80. The error correcting circuit 80 corrects an error of the output of the demodulator 79. When necessary, the error correcting circuit 80 interpolates an error that cannot be corrected. The error correcting circuit 80 outputs reproduction data to an input terminal of a switch 81. The switch 81 outputs a PCM audio signal as reproduced AV information and reproduced living body information to output terminals 82a and 82b, respectively.

TOC recorded in a lead-in area of the optical disc 71 contains the same information as the TOC of a CD and address information of a boundary position of a recording region of living body information and a recording region of AV information. When the optical disc 71 is loaded into the reproducing apparatus, the lead-in area is read as a reading position. As a result, the TOC is read. Like the conventional CD, the TOC reading circuit 83 causes the total number of music programs, the total play time, and so forth to be displayed in accordance with the TOC that has been read. When living body information and AV information have been recorded on the optical disc, a region information detecting circuit 84 detects the address information of the boundary thereof.

The address information of the boundary of the two regions detected by the region information detecting circuit 84 is supplied to the region controlling portion 85. A reproduction address is supplied from the sub code detecting circuit 77 to the region controlling circuit 85. The region controlling portion 85 is connected to the controller 78. The region controlling portion 85 compares the reproduction address corresponding to the reproduction position of the optical disc 71 and the address information of the boundary detected by the region information detecting circuit 84 and outputs a control signal that causes the switch 81 to change the switch position to another switch position when they match. Thus, when data is reproduced from the inner periphery of the optical disc 71, a PCM audio signal is reproduced from a session P1 and output to an output terminal 82a. Thereafter, living body information is reproduced from a session P2 and output to an output terminal 82b.

The foregoing reproducing apparatus successively reads data from the sessions of the optical disc 71 with one optical pickup. Alternatively, living body information may be pre-read and stored in a memory. Alternatively, AV information and living body information may be simultaneously reproduced with two pickups.

Video information and audio information of reproduction AV information obtained from the output terminal 82a are reproduced by a display, a projector, or the like and a speaker, respectively. Living body information of the performer obtained from the output terminal 82b is reproduced as at least one of a sound, a picture, and a vibration that vary. For example, when a chair on which the listener sits vibrates in accordance with an input signal, the chair is vibrated in accordance with living body information.

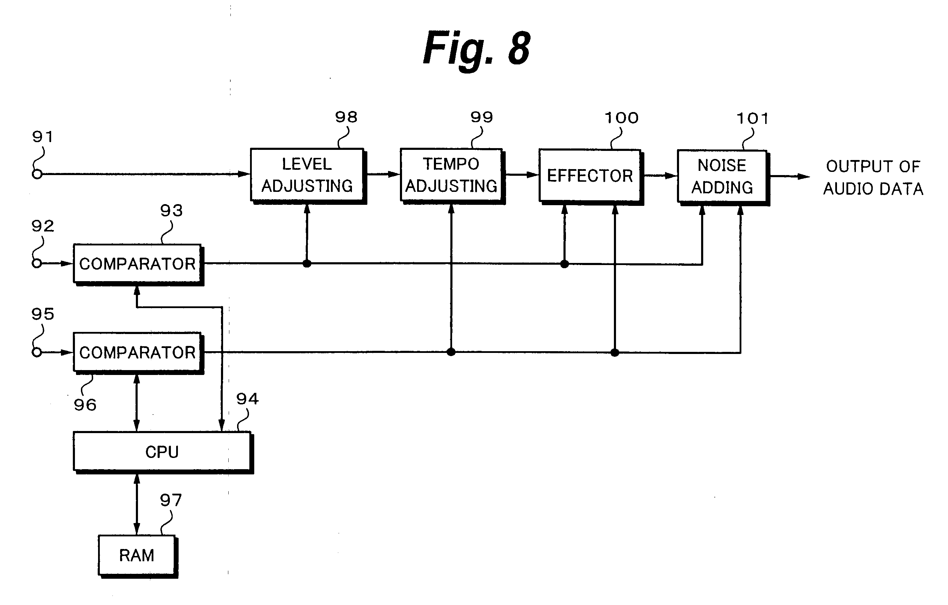

Alternatively, reproduction AV information may be affected in accordance with living body information of the performer. Fig. 8 shows an example of a structure of a reproduction control. In Fig. 8, reference numeral 91 represents an input terminal to which reproduction AV information for example a PCM audio signal is input. Reference numeral 92 represents an input terminal of living body information for example a breath depth signal (obtained at the output terminal 7a of_Fig. 2) of the breath signal. Reference numeral 95 represents an input terminal of a breath interval signal (obtained at the output terminal 7c of Fig. 2). Start timings of the PCM audio signal, the breath depth signal, and the breath interval signal are matched. In other words, these signals are temporally synchronized and input.

The breath depth signal is input to a comparator 93. A reference signal is supplied from a controller 94 to the comparator 93. The comparator 93 compares the breath depth signal with the reference signal. The breath interval signal is input to a comparator 96. A reference signal is supplied from the controller 94 to the comparator 96. The comparator 96 compares the breath interval signal with the reference signal. The reference signals are stored in a database composed of a RAM 97. The controller 94 selectively reads a reference signal from the RAM 97 and supplies the reference signal to the comparator 93. Likewise, a reference signal supplied to the comparator 96 is read from the RAM 97 by the controller 94.

An example of a reference signal stored in the RAM 97 is standard data about the performer. When AV information is music, a plurality of sets of standard data is pre-stored in accordance with a conductor, singer (male or female), genre of music, and so forth. Standard data is automatically selected in accordance with a switch operation of the viewer/listener or identification information recorded in the TOC.

The comparator 93 generates a binary comparison output that represents whether or not the breath depth signal of the performer is larger than standard data. Likewise, the comparator 96 generates a binary comparison output that represents whether the breath interval signal of the performer is larger than standard data. Instead of the comparators, dividing circuits may be disposed so that a signal of breath information is standardized in accordance with a reference signal. In this case, a normalized output is generated instead of a binary output.

The PCM audio signal that is input from the input terminal 91 to a level adjusting circuit 98. The level adjusting circuit 98 controls the level of the PCM audio signal in accordance with the comparison output of the comparator 93. When the breath depth signal represents that the breathing becomes deep, the level adjusting circuit 98 increases the level of the PCM audio signal. An output signal of the level adjusting circuit 98 is supplied to a tempo adjusting circuit 99.

The tempo adjusting circuit 99 controls the tempo of the PCM audio signal in accordance with the comparison output of the comparator 96. For example, the breath interval signal and the tempo are synchronized. When the breath intervals become short, the tempo adjusting circuit 99 increases the tempo. An output signal of the tempo adjusting circuit 99 is supplied to an effecter 100.

The effecter 100 is controlled in accordance with output signals of the comparators 93 and 96. The effecter 100 controls a frequency component of the PCM audio signal in accordance with the breath depth and the breath intervals. An output signal of the effecter 100 is supplied to a noise adding circuit 101.

The noise adding circuit 101 is controlled by the output signals of the comparators 93 and 96. A level noise and an interval noise of the breath depth and the breath intervals are detected. The noise adding circuit 101 adds noises in accordance with the detected noises. The noises are 1/fⁿ noises (where n is any integer larger than 0). The level of the PCM audio signal is controlled in accordance with the level noise. The tempo of the PCM audio signal is controlled in accordance with the interval noise. An output signal is obtained from the noise adding circuit 101. The structure shown in Fig. 8 is an example. Thus, it is not necessary to perform the control in accordance with all the living body information shown in Fig. 8.

Next, another embodiment of the present invention will be described. According to the present embodiment, when the AV information is recorded or transmitted, AV information is frame-segmented, block-segmented, or packetized. Living body information is contained in each transmission unit.

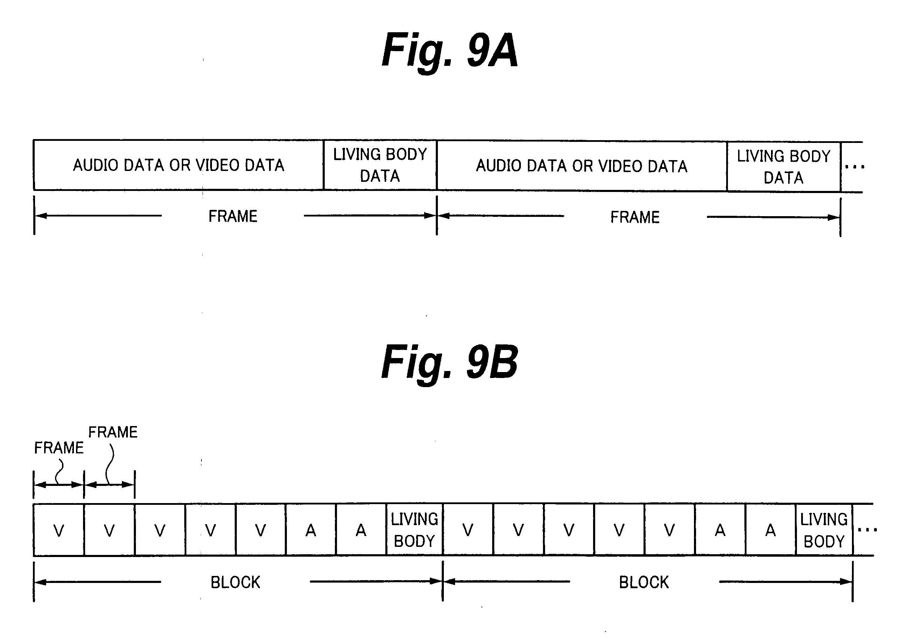

Fig. 9A shows a frame structure according to the present invention. One frame contains AV data and living body data. Fig. 9B shows a block structure according to the present invention. One block contains a plurality of frames. A frame of video data (represented by V) and a frame of audio data (represented by A) are multiplexed on time division basis. For example, five video data frames, two audio data frames, and one living body information frame compose one block. Instead of frame-segmentation and block-segmentation, AV information may be packetized.

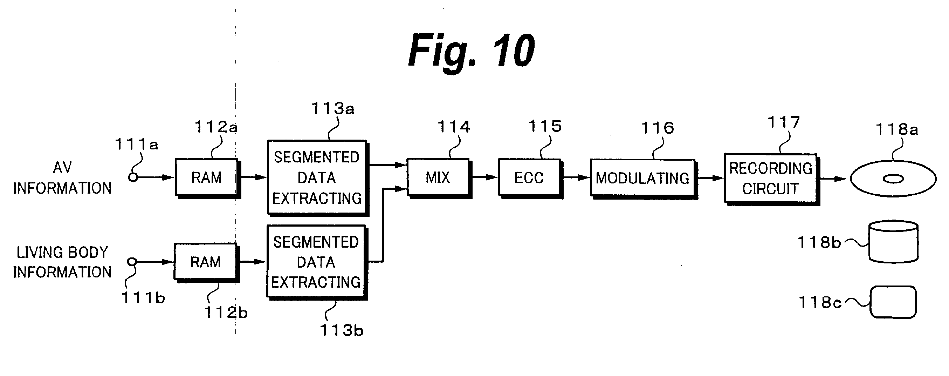

Fig. 10 shows a structure of a recording apparatus according to another embodiment. In Fig. 10, reference numeral 111a represents an input terminal for AV information. Reference numeral 111b represents an input terminal for living body information of the performer temporally synchronized with the AV information. The input AV data and living body data are stored in RAMs 112a and 112b as buffer memories, respectively. Segmented data extracting circuits 113a and 113b are connected to the RAMs 112a and 112b, respectively.

The segmented data extracting circuit 113a extracts AV data for a data amount corresponding to a segmented data unit for example a frame. The segmented data extracting circuit 113b extracts living body data for a data amount in accordance with a segmented data unit for example a frame. Output data of the segmented data extracting circuits 113a and 113b is supplied to a mixing circuit 114. As shown in Fig. 9A, data of which AV data and living body information are segmented as a frame is output from the mixing circuit 114.

An output signal of the mixing circuit 114 is supplied to a modulating circuit 116 through an error correction code encoder 115. Data that has been encoded with an error correction code and modulated is supplied to a recording circuit 117. A record signal of the output of the recording circuit 117 is recorded on an optical disc 118a. In this case, an optical pickup (not shown) is used. A structure similar to that for the signal process for the optical disc 118a can be used to record a record signal to a hard disk drive 118b or an optical card 118c.

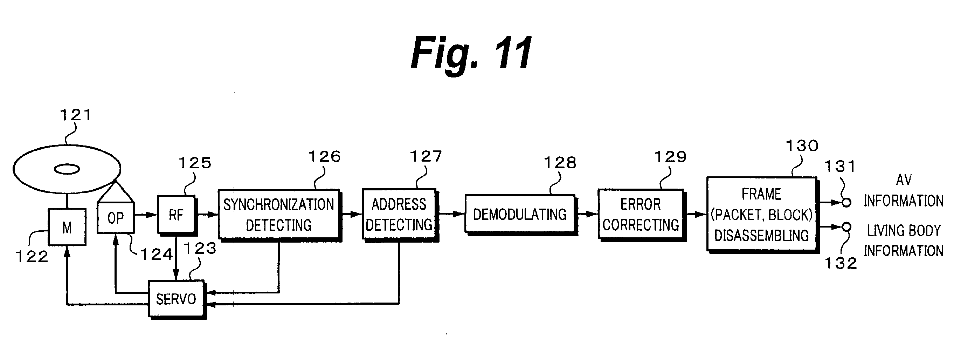

Next, with reference to Fig. 11, a data reproducing apparatus that reproduces data from a data recording medium on which the data was recorded by the data recording apparatus shown in Fig. 10 will be described. As described above, in the data recording medium from which data is reproduced, AV information and living body information of the performer that are temporally synchronized are segmented. In Fig. 11, reference numeral 121 represents an optical disc on which AV information and living body information that are segmented as shown in Fig. 9A or Fig. 9B are recorded by the recording apparatus shown in Fig. 10.

An optical disc 121 is placed on a turn table and rotated by a spindle motor 122. The spindle motor 122 is driven and rotated at a constant linear velocity (CLV) under the control of a servo portion 123. The servo portion 123 generates various types of servo drive signals for focus, tracking, thread, and spindle servo drives in accordance with a focus error signal, a tracking error signal, and an operation command received from a controller (not shown) and outputs these signals to the spindle motor 122 and an optical pickup 124. The optical pickup 124 focuses a light beam of a semiconductor laser on a signal side of the optical disc 121 and traces tracks formed in a concentrically circular shape or spiral shape on the optical disc 121. All the optical pickup 124 is moved by a thread mechanism.

An output of the optical pickup 124 is supplied to the synchronization detector 126 through an RF amplifier 125. An output of the synchronization detector 126 is supplied to an address detecting circuit 127. The synchronization detector 126 detects a synchronization signal such as a frame synchronization signal of a reproduction signal. The address detecting circuit 127 detects an address signal of the optical disc 121. A servo signal that is output from the RF amplifier 125 is supplied to the servo portion 123. An address signal detected by the address detecting circuit 127 is supplied to the servo portion 123 and a controller (not shown).

An output signal of the address detecting circuit 127 is supplied to a demodulator 128. An output of the demodulator 128 is supplied to an error correcting circuit 129. The error correcting circuit 129 corrects an error of the reproduction data. When necessary, the error correcting circuit 129 interpolates an error that cannot be corrected. The reproduction data that is output from the error correcting circuit 129 is supplied to a frame disassembling circuit 130. The frame disassembling circuit 130 separates one frame into AV information and living body information and outputs them to output terminals 131 and 132, respectively. When the reproduction data is a packet or a block, the frame disassembling circuit 130 separates the packet or block into the output terminals 131 and 132, respectively.

Like the foregoing embodiment, living body information of the performer that is obtained from the output terminal 132 is reproduced as at least one of a sound, a picture, and a vibration that vary. When a chair on which the viewer/listener sits vibrates in accordance with an input signal, the chair is vibrated in accordance with living body information. Alternatively, as described with reference to Fig. 8, reproduction AV information can be controlled in accordance with living body information of the performer.

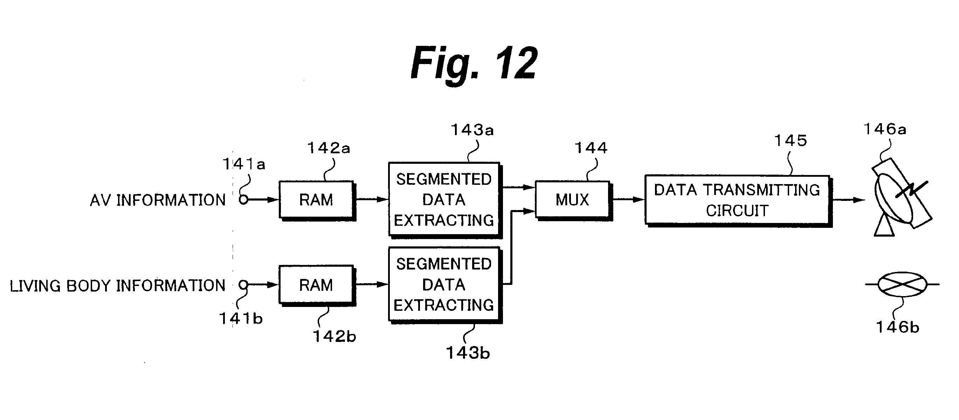

Fig. 12 shows a structure of a data transmitting apparatus according to another embodiment of the present invention. Reference numeral 141a represents an input terminal for AV information. Reference numeral 141b represents an input terminal for living body information of the performer that is temporally synchronized with AV information. The input AV data and living body data are stored in RAMs 142a and 142b as buffer memories. Segmented data extracting circuits 143a and 143b are connected to the RAMs 142a and 142b, respectively.

The segmented data extracting circuit 143a extracts AV data for a data amount corresponding to a segmented data unit for example a packet. The segmented data extracting circuit 143b extracts living body data for a data amount in accordance with a segmented data unit for example a packet. Output data of the segmented data extracting circuits 143a and 143b is supplied to a multiplexer 144. As shown in Fig. 9B, the multiplexer 144 outputs one frame of AV data and living body data.

An output signal of the multiplexer 144 is supplied to a data transmitting circuit 145. The data transmitting circuit 145 performs an error correction code encoding process, a modulating process, and so forth for the output signal of the multiplexer 144. A transmission signal that is output from the data transmitting circuit 145 is supplied to a transmission antenna 146a and transmitted as a radio wave or to a network 146b.

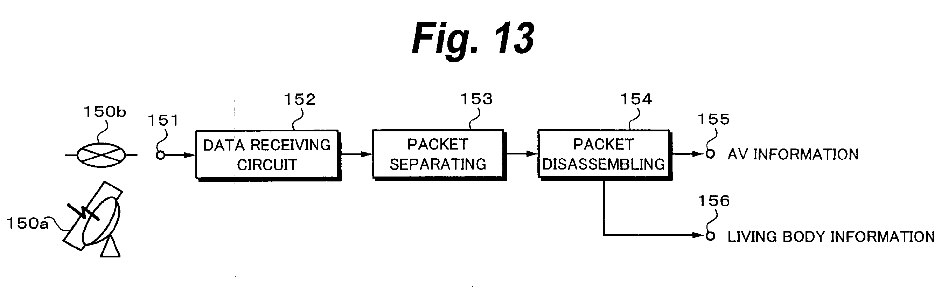

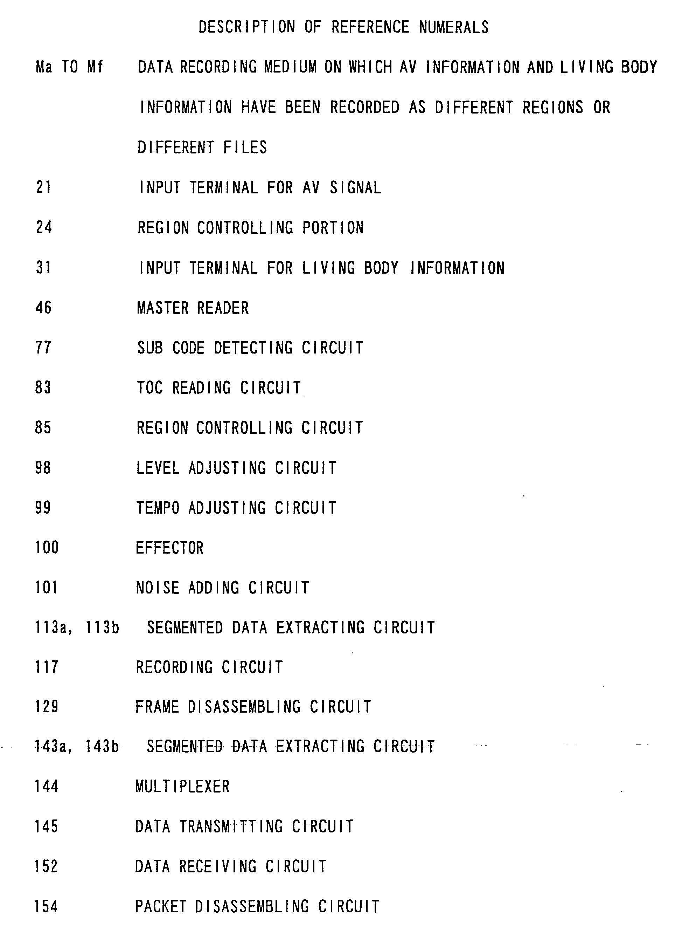

Next, with reference to Fig. 13, a data receiving apparatus that receives data transmitted by the data transmitting apparatus shown in Fig. 12 will be described. As described above, received data contains AV information and living body information of the performer that are temporally synchronized and segmented. In Fig. 13, reference numeral 150a represents a receiving antenna that receives data of which AV information and living body information are segmented as shown in Fig. 9A or Fig. 9B and transmitted by the transmitting apparatus shown in Fig. 10. Reference numeral 150b represents a network that receives data from the transmitting apparatus shown in Fig. 10.

Reception data received through the receiving antenna 150a or the network 150b is supplied from an input terminal 151 to a data receiving circuit 152. The data receiving circuit 152 performs a demodulating process, an error correcting process, and so forth for the reception data and obtains reception data of which a packet of AV information and a packet of living body information are multiplexed on time division basis.

The reception data is supplied to a packet separating circuit 153. The packet separating circuit 153 separates data packets from the reception data. The separated data packets are supplied to a packet disassembling circuit 154. The packet disassembling circuit 154 disassembles the data packets into packets of AV information and packets of living body information. AV information is output to one output terminal 155. Living body information is output to another output terminal 156. The reception AV information is reproduced as a stream by for example a personal computer.

Like the foregoing embodiment, living body information of the performer is reproduced as at least one of a sound, a picture, and a vibration that vary. In addition, as described with reference to Fig. 8, reproduction AV information may be controlled in accordance with living body information of the performer.

Although the present invention has been shown and described with respect to a best mode embodiment thereof, it should be understood by those skilled in the art that the foregoing and various other changes, omissions, and additions in the form and detail thereof may be made therein without departing from the spirit and scope of the present invention. For example, the brightness of lighting may be varied in accordance with living body information of the performer. Alternatively, the level, tempo, and so forth of a very low frequency sound may be controlled in accordance with living body information of the performer. In addition, living body information of an actor in a leading role of a stage or a movie or living body information of a pantomime performer may be detected and recorded or transmitted along with a picture thereof.

As is clear from the foregoing description, according to the present invention, at least one of audio information and video information is recorded or transmitted along with living body information of a music performer, an actor, and/or audience. When the recorded or transmitted data is reproduced, the living body information is reproduced as a vibration or the like. Alternatively, reproduction of AV information is controlled in accordance with the living body information. As a result, a live power can be transmitted to the viewer/listener. Thus, at least one of audio information and video information can be reproduced as if the viewer/listener were present in a concert hall.

## Claims

1. A data recording medium having a first region and a second region, at least one of audio data and video data being recorded in the first region, living body information in relation to the data being recorded in the second region.

2. The data recording medium as set forth in claim 1,
wherein the audio information is one of linear PCM data, compressed audio data, and one-bit digital audio.

3. The data recording medium as set forth in claim 1,
wherein the video information is a digital video.

4. The data recording medium as set forth in claim 3,
wherein the digital video has been compression-encoded.

5. The data recording medium as set forth in claim 1,
wherein the data chronologically synchronizes with the living body information.

6. The data recording medium as set forth in claim 1,
wherein the living body information is information that represents at least one of a body motion, a biochemical reaction, a brain wave, a magnetoencephalography, an electromyogram, a body surface temperature, perspiration of a skin, a skin resistance, a pulsation, breath, a micro vibration, an electrocardiogram, a heartbeat, and a blood pressure.

7. The data recording medium as set forth in claim 1,
wherein the living body information is living body information of a person or people who perform at least one of the audio data and the video data.

8. The data recording medium as set forth in claim 1,
wherein the living body information is living body information of a person or people who at least either listen to the audio data or watch the video data.

9. The data recording medium as set forth in claim 1,
wherein the living body information is information that represents at least one of a peak position, a peak interval, a peak level, and a varied value of a living body signal.

10. The data recording medium as set forth in claim 1,
wherein the data recording medium is a magnetic tape, an optical disc, a magnetic disc, a semiconductor memory, an organic compound memory, an optical card, or a magnetic card.

11. The data recording medium as set forth in claim 1,
wherein the first region and the second region are one of combinations of an inner peripheral portion and an outer peripheral portion, a first layer and a second layer, a first half portion and a second half portion, and a first file and a second file.

12. The data recording medium as set forth in claim 1, further comprising:
a management region in which management information is recorded, address information that represents a boundary position of the first region and the second region being recorded in the management region.

13. A recording method for recording at least one of audio data and video data to a first region and living body information in relation to the data to a second region.

14. A recording apparatus for recording at least one of audio data and video data to a first region and living body information in relation to the data to a second region.

15. A reproducing method, comprising the steps of:
reading data from a data recording medium having a first region and a second region, at least one of audio data and video data being recorded in the first region, living body information in relation to the data being recorded in the second region; and
reproducing the data that has been read and reproducing the living body information with one of a sound, a picture, and a vibration.

16. A reproducing method, comprising the steps of:
reading data from a data recording medium having a first region and a second region, at least one of audio data and video data being recorded in the first region, living body information in relation to the data being recorded in the second region; and
controlling the reproduction of the data recorded in the first region corresponding to the living body information.

17. A reproducing apparatus, comprising:
reading means for reading data from a data recording medium having a first region and a second region, at least one of audio data and video data being recorded in the first region, living body information in relation to the data being recorded in the second region;
reproducing means for reproducing the data that has been read by the reading means; and
reproduction controlling means for reproducing the data that is recorded in the first region and reproducing the living body information, which is in relation to the data, with one of a sound, a picture, and a vibration.

18. A reproducing apparatus, comprising:
reading means for reading data from a data recording medium having a first region and a second region, at least one of audio data and video data being recorded in the first region, living body information in relation to the data being recorded in the second region;
reproducing means for reproducing the data that has been read by the reading means; and
controlling means for controlling the reproduction of the data recorded in the first region corresponding to the living body information.

19. The reproducing apparatus as set forth in claim 18,
wherein the reading means is configured to read a plurality of pieces of the living body information from the data recording medium, and
wherein the controlling means is configured to select a predetermined one of the plurality of pieces of the living body information and control the reproduction of the data recorded in the first region corresponding to the selected piece of the living body information.

20. The reproducing apparatus as set forth in claim 18,
wherein the controlling means is configured to control a reproduction speed or a reproduction level of the data recorded in the first region corresponding to the living body information.

21. A data transmitting method, comprising the steps of:
dividing at least one of audio data and video data in a predetermined region;
generating block data composed of the divided data and living body information corresponding to the predetermined region; and
transmitting the generated block data.

22. The data transmitting method as set forth in claim 21,
wherein the predetermined region is in the unit of a frame, a block, a sector, or a predetermined time period.

23. The data transmitting method as set forth in claim 21,
wherein the audio data is one of linear PCM data, compressed audio data, and one-bit digital audio.

24. The data transmitting method as set forth in claim 21,
wherein the video data is digital video data.

25. The data transmitting method as set forth in claim 21,
wherein the digital video has been compression-encoded.

26. The data transmitting method as set forth in claim 21,
wherein the living body information is information that represents at least one of a body motion, a biochemical reaction, a brain wave, a magnetoencephalography, an electromyogram, a body surface temperature, perspiration of a skin, a skin resistance, a pulsation, breath, a micro vibration, an electrocardiogram, a heartbeat, and a blood pressure.

27. The data transmitting method as set forth in claim 21,
wherein the living body information is living body information of a person or people who perform at least one of the audio data and the video data.

28. The data transmitting method as set forth in claim 21,
wherein the living body information is living body information of a person or people who at least either listen to the audio data or watch the video data.

29. The data transmitting method as set forth in claim 21,
wherein the living body information is information that represents at least one of a peak position, a peak interval, a peak level, and a varied value of a living body signal.

30. A data transmitting apparatus, comprising:
dividing means for dividing at least one of audio data and video data in a predetermined region;
generating means for generating block data composed of the divided data divided by the dividing means and living body information corresponding to the predetermined region; and
transmitting means for transmitting the block data generated by the generating means.

31. A recording method, comprising the steps of:
dividing at least one of audio data and video data in a predetermined region;
generating block data composed of the divided data and living body information corresponding to the predetermined region; and
recording the generated block data.

32. A recording apparatus, comprising:
data dividing means for dividing at least one of audio data and video data in a predetermined region;
generating means for generating block data composed of the divided data and living body information corresponding to the predetermined region; and
recording means for recording the block data generated by the generating means to a recording medium.

33. A recording medium on which at least one of audio data and video data divided in a predetermined region and living body information corresponding to the predetermined region are recorded as a block.

34. A reproducing method, comprising the steps of:
receiving a block composed of data and living body information, the data being at least one of audio data and video data; and
reproducing the data and reproducing the living body information with one of a sound, a picture, and a vibration.

35. A reproducing method, comprising the steps of:
receiving block data composed of data and living body information, the data being at least one of audio data and video data; and
controlling the block data corresponding to the living body information and reproducing the block data.

36. A reproducing method, comprising the steps of:
reading block data from a recording medium, the block being composed of data and living body information, the data being at least one of audio data and video data divided in a predetermined region, the living body information corresponding to the predetermined region; and
reproducing the data and reproducing the living body information with one of a sound, a picture, and a vibration.

37. A reproducing method, comprising the steps of:
reading block data composed of data and living body information, the data being at least one of audio data and video data divided in a predetermined region, the living body information corresponding to the predetermined region; and
controlling the data corresponding to the living body information and reproducing the data.

38. A reproducing apparatus, comprising:
receiving means for receiving block data composed of data and living body information, the data being at least one of audio data and video data divided in a predetermined region, the living body information corresponding to the predetermined region; and
reproducing means for reproducing the data and reproducing the living body information with one of a sound, a picture, and a vibration.

39. A reproducing apparatus, comprising:
receiving means for receiving block data composed of data and living body information, the data being at least one of audio data and video data divided in a predetermined region, the living body information corresponding to the predetermined region;
reproducing means for reproducing the block data; and
controlling means for reproducing the data corresponding to the living body information.

40. A reproducing apparatus, comprising:
reading means for reading block data from a recording medium, the block data being composed of data and living body information, the data being at least one of audio data and video data divided in a predetermined region, the living body information corresponding to the predetermined region; and
reproducing means for reproducing the data and reproducing the living body information with one of a sound, a picture, and a vibration.

41. A reproducing apparatus, comprising:
reading means for reading block data from a recording medium, the block data being composed of data and living body information, the data being at least one of audio data and video data divided in a predetermined region, the living body information corresponding to the predetermined region;
reproducing means for reproducing the data; and
controlling means for controlling the reproduction of the data corresponding to the living body information.
